# EUROPÄISCHE PATENTSCHRIFT

(11) **EP 2 775 919 B1**
(45) Veröffentlichungstag und Bekanntmachung des Hinweises auf die Patenterteilung: **05.08.2015**
(21) Anmeldenummer: 12783995.9
(22) Anmeldetag: 08.11.2012
(51) Int. Cl.: A61B 5/15, A61B 5/151

(54) **VERFAHREN ZUM HERSTELLEN VON ANALYTISCHEN HILFSMITTELN**
METHOD FOR PRODUCING ANALYTICAL AIDS
PROCÉDÉ DE FABRICATION D'ACCESSOIRES D'ANALYSE

(30) Priorität: 10.11.2011 EP 11188643
(43) Veröffentlichungstag der Anmeldung: 17.09.2014
(73) Patentinhaber: Roche Diagnostics GmbH, 68305 Mannheim (DE); F.Hoffmann-La Roche AG, 4070 Basel (CH)
(72) Erfinder: IHLE, Günther, 69526 Mauer (DE); LIST, Hans, 64754 Hesseneck-Kailbach (DE)
(74) Vertreter: Pfiz, Thomas
(86) Internationale Anmeldenummer: PCT/EP2012/072113
(87) Internationale Veröffentlichungsnummer: WO 2013/068447

(56) Entgegenhaltungen:
- EP-A1- 0 038 297
- WO-A1-2006/066744
- DE-A1- 10 225 451
- DE-A1-102007 056 112
- DE-C1- 10 034 806
- US-A- 4 100 393
- US-A- 5 515 871
- US-A- 5 889 255

## Beschreibung

Die Erfindung betrifft ein Verfahren zum Herstellen von analytischen Hilfsmitteln zum Gewinnen von Körperflüssigkeit, insbesondere von Lanzetten oder Microsamplern, bei dem die Hilfsmittel als Formteil aus einem metallischen Flachmaterial unter Materialtrennung an Formteilkanten gebildet werden. Die Erfindung betrifft weiter ein nach einem solchen Verfahren hergestelltes analytisches Hilfsmittel zum Gewinnen von Körperflüssigkeit.

Im Bereich der Diagnostik ist es in vielen Fällen notwendig, Proben von Körperflüssigkeit, insbesondere Blutproben oder Proben interstitieller Flüssigkeit, zu gewinnen, um darin Inhaltsstoffe, insbesondere bestimmte Analyte, nachweisen zu können. Beispiele derartiger Analyte sind Blutglukose, Gerinnungsparameter, Triglyceride, Lactat oder ähnliches. Entsprechend der nachgewiesenen Konzentrationen kann dann beispielsweise über eine entsprechende Behandlung entschieden werden. Dabei werden in der Regel ein oder mehrere analytische Hilfsmittel als Einwegteil von einem Probanden selbst benutzt, um die Proben an Körperflüssigkeit zu gewinnen und/oder zu analysieren. So können die analytischen Hilfsmittel beispielsweise Lanzetten umfassen, welche zur Ausführung einer Stechbewegung antreibbar sind, um eine Öffnung in der Haut des Probanden zu erzeugen, durch welche die Körperflüssigkeit entnommen werden kann. Daneben sind auch integrierte Testelemente bekannt, welche zum Sammeln der Körperflüssigkeitsprobe als auch dem Probentransport und gegebenenfalls sogar zur qualitativen und/oder quantitativen Analyse dieser Probe dienen. Beispiele derartiger analytischer Hilfsmittel sind so genannte Microsampler, bei denen mittels einer Lanzette ein Einstich oder Einschnitt erzeugt wird, die Probe aufgenommen wird und zu einem oder mehreren Testfeldern transportiert wird.

Diese Testfelder können separat von der Lanzette angeordnet sein, können jedoch auch Bestandteil der Lanzette selbst sein.

Eine technische Herausforderung bei der Bereitstellung analytischer Systeme und analytischer Hilfsmittel besteht jedoch darin, diese als kostengünstiges Massenprodukt unter geeigneten Bedingungen in gleichbleibend hoher Qualität bereitzustellen. Dabei wurde beispielsweise in der WO 2006/066744 A1 schon vorgeschlagen, zumindest die Grundkörper der disposiblen Hilfsmittel durch Maskenätzen als Formätzteil aus einem metallischen Flachmaterial zu bilden. Der Ätzprozess sorgt allerdings dafür, dass die Teilekanten extrem scharf werden. An der Lanzettenspitze ist dies erwünscht, während scharfe Hinterkanten im Haltebereich zum Hindernis bei der Ausführung des Stechvorgangs werden können. Hierbei können bei der hin und her gehenden Stechbewegung innerhalb einer Führung oder Magazinkammer Störkonturen auftreten und/oder durch Materialabrieb störende Partikel entstehen, welche die Analyse verfälschen bzw. die Sammelstrukturen blockieren.

Ausgehend hiervon liegt der Erfindung die Aufgabe zugrunde, die im Stand der Technik bekannten Herstellungsverfahren und entsprechend hergestellte analytische Hilfsmittel weiter zu verbessern und insbesondere mit geringem Fertigungsaufwand für eine einwandfreie Funktion zu sorgen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen. Es werden ein Verfahren zur Herstellung eines analytischen Hilfsmittels sowie ein analytisches Hilfsmittel vorgeschlagen, wobei das analytische Hilfsmittel unter Verwendung eines erfindungsgemäßen Herstellungsverfahrens herstellbar sein kann. Dementsprechend kann bezüglich möglicher Ausgestaltungen des analytischen Hilfsmittel auf die Beschreibung des Verfahrens verwiesen werden und umgekehrt.

Die Erfindung geht von dem Gedanken aus, durch eine mehrspurige Laserbestrahlung eine gleichmäßige Kantenverrundung von störenden Formteilkanten zu erzielen. Dementsprechend wird erfindungsgemäß vorgeschlagen, dass mindestens eine scharfkantige Formteilkante durch Laserbestrahlung nachbearbeitet wird, wobei ein Laserstrahl entlang einer Bestrahlungsbahn in Bestrahlungsintervallen mehrfach über das Formteil hinweg geführt wird und die Formteilkante durch den akkumulierten Energieeintrag des Laserstrahls verrundet wird. Damit ist es möglich, gezielt Strukturbereiche auch in mikroskopischen Abmessungen und komprimierter Anordnung zu bearbeiten, ohne die Werkstücke bzw. Bauteile in der makroskopischen Form zu verändern. Der Energieeintrag in den einzelnen Bestrahlungsintervallen kann entsprechend gering eingestellt werden, wobei Wärme von der Bauteilseite her gleichsam schonend an die zu bearbeitende Kante herangeführt wird, so dass nur die schneidhaltige Kantenschärfe beseitigt wird.

Überraschend hat sich gezeigt, dass die Kantenverrundung optimiert wird, wenn der Laserstrahl ausgehend von einem kantenfernen Materialbereich des Formteils in einen kantennahen Bereich geführt wird. Bevorzugt wird der Laserstrahl ausgehend von innerhalb der Formteiloberfläche her in seitlich nebeneinander liegenden Spuren auf die Formteilkante zu geführt.

Weitere Verbesserungen lassen sich erzielen, wenn der Laserstrahl in seitlich einander überlappenden Spuren geführt wird, und wenn der Laserstrahl intervallweise mit abnehmendem seitlichem Versatz zur Formteilkante geführt wird.

Allgemein sollte der Laserstrahl in einem seitlichen Abstand entlang der Formteilkante und vorzugsweise parallel dazu geführt werden, wobei der seitliche Abstand der gegebenenfalls auch bogenförmigen Laserspur zur Formteilkante geringfügig variieren kann.

Für eine herstellungstechnische Vereinfachung insbesondere auch im Hinblick auf eine Magazinierung ist es von Vorteil, wenn die zu bearbeitenden Formteilkanten einer Vielzahl von Formteilen kreisförmig angeordnet werden, und wenn der Laserstrahl auf einer Spiralbahn oder in konzentrischen Kreisen über die Formteile hinweg zu den zu entgratenden Formteilkanten hin geführt wird. Alternativ ist es auch möglich, dass die zu entgratenden Formteilkanten einer Vielzahl von Formteilen linear angeordnet werden, und dass die nebeneinander liegenden Formteile mit dem Laserstrahl zeilenweise überstrichen werden.

Für eine mikroskopische Formteilgestaltung insbesondere in einer Massenfertigung ist es vorteilhaft, wenn eine Vielzahl von Formteilen in sternförmiger oder linearer Anordnung gegebenenfalls über Materialbrücken zusammenhängend in einem einheitlichen Ätzvorgang aus einem Flachmaterialstück gebildet werden.

Vorteilhafterweise werden die Hilfsmittel durch Maskenätzen als Formteil gebildet. Dabei ist es für bestimmte Formbereiche wie insbesondere Lanzettenspitzen vorteilhaft, wenn beim Maskenätzen der Formteile unter der Einwirkung eines Ätzmittels durch Unterätzen einer Formteilmaske eine gegebenenfalls gratbehaftete, scharfe Formteilkante gebildet wird. Dementsprechend können die Formteile mit einer scharfen Spitze zum Einstechen in die Haut und gegebenenfalls einem Sammelkanal für Körperflüssigkeit im Bereich der Spitze geformt werden.

Um Störkonturen zu vermeiden, ist es von besonderem Vorteil, wenn eine Formteilkante in einer zur Antriebskopplung ausgebildeten Kopplungspartie der Formteile verrundet wird.

Zur Vermeidung von Strukturschäden ist es vorteilhaft, wenn der Energieeintrag des Laserstrahls in den einzelnen Bestrahlungsintervallen unterhalb der Schwelle zu einem Materialabtrag eingestellt wird.

Vorteilhafterweise wird die Anzahl der Bestrahlungsintervalle nach Maßgabe eines gewünschten Kantenmaßes einer Formteilkante vorbestimmt. Besonders günstig ist es, wenn die Formteilkante mit einem Kantenradius von mehr als 30 µm, vorzugsweise etwa 50 µm verrundet wird.

Gegenstand der Erfindung ist auch ein analytisches Hilfsmittel zum Gewinnen von Körperflüssigkeit, mit einem durch Maskenätzen aus einem metallischen Flachmaterial gebildeten Formteil, wobei mindestens eine Formteilkante durch Laserbestrahlung in einer mehrspurigen Bestrahlungsbahn verrundet ist.

Im Folgenden wird die Erfindung anhand der in der Zeichnung schematisch dargestellten Ausführungsbeispiele näher erläutert. Es zeigen
- Fig. 1: ein als Microsampler ausgebildetes analytisches Verbrauchsmittel in einer perspektivischen Darstellung;
- Fig. 2: einen proximalen Endabschnitt des durch Maskenätzen geformten Microsamplers im Längsschnitt;
- Fig. 3: eine Vorrichtung zur Lasernachbearbeitung des Microsamplers in schaubildlicher Darstellung; distalen rückseitigen Maskenabschnitt in einer Fig. 1 entsprechenden Darstellung;
- Fig. 4: ein weiteres Ausführungsbeispiel von aus einem Blechstück geätzten Microsamplern in perspektivischer Ansicht; und
- Fig. 5: eine mehrspurige Laserbestrahlungsbahn zur proximalen Kantenverrundung in einem Ausschnitt der Fig. 4.

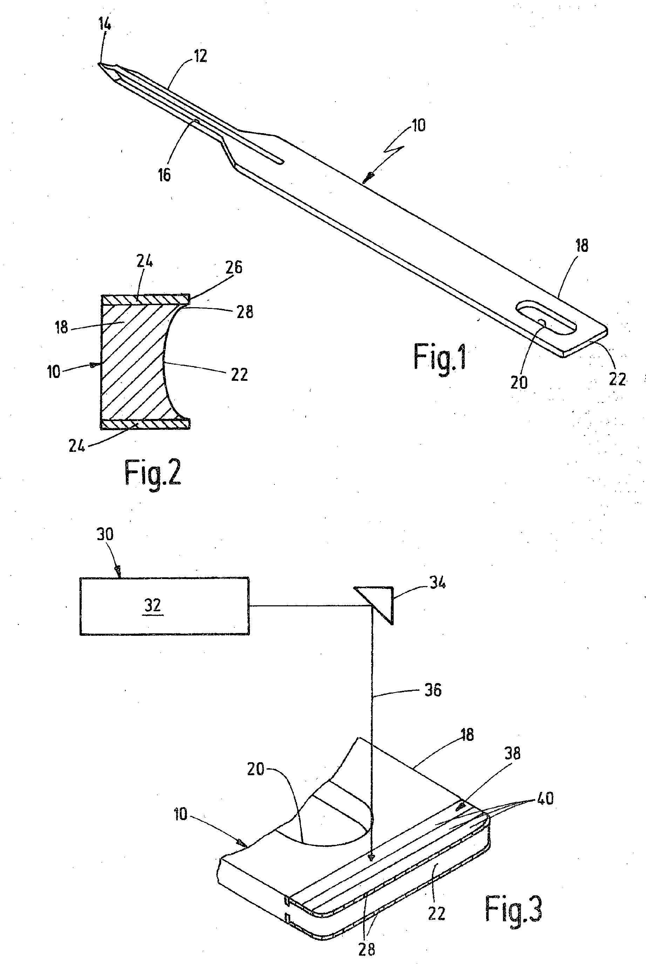

Das in Fig. 1 dargestellte Stech- und Sammelelement 10 ermöglicht als sogenannter Microsampler das direkte Sammeln von Körperflüssigkeit (Blut, Gewebeflüssigkeit) durch einen mikroskopischen Hauteinstich zum Nachweis eines Analyten, beispielsweise Glukose in einem nicht gezeigten Handgerät. Zu diesem Zweck weist der Microsampler 10 einen Stechschaft 12 mit einer distalen Spitze 14 und einem rillenförmigen Sammelkanal 16 für Körperflüssigkeit sowie einen proximalen Halteteil 18 mit einer Kopplungspartie 20 zur Antriebskopplung eines Stechantriebs des Handgeräts für die Ausführung einer Stechbewegung. Um hierbei Störkonturen zu vermeiden, ist eine Lasernachbearbeitung der durch Maskenätzen geformten Microsampler 10 im Bereich einer proximalen Hinterkante 22 vorgesehen, wie es weiter unten erläutert wird.

Ein solcher Microsampler 10 lässt sich als integriertes diagnostisches Verbrauchsmittel mit einem Testelement zur einmaligen Bestimmung des Analyten kombinieren. Grundsätzlich betrifft das erfindungsgemäße Verfahren auch die Herstellung von Lanzetten, die nur zur Erzeugung einer Stichwunde in der Haut eines Probanden ausgelegt sind.

Wie in Fig. 2 veranschaulicht, lassen sich Microsampler 10 als Formätzteile durch Maskenätzen aus einem metallischen Substrat bzw. Flachmaterial herstellen. Dabei wird eine Ätzmaske 24 als doppelseitiges Layout auf die beiden Seiten eines dünnen Edelstahl-Substrats aufgebracht. Die Ätzmaske 24 kann durch Fotolithografie, d.h. durch Belichten und Auswaschen aus Fotolack in an sich bekannter Weise auf dem Substrat strukturiert werden. Über die Freisparungen in der so erzeugten Ätzmaske 24 erfolgt anschließend eine Beaufschlagung des Substrats mit einem Ätzmittel, wobei die abgedeckten bzw. maskierten Bereiche der Grundform nach freigeätzt werden und anschließend die Ätzmaske 24 entfernt wird. Hierbei ist zu berücksichtigen, dass der Materialabtrag nicht nur in die Tiefe, sondern auch durch Hinterätzen von Randkonturen 26 der Ätzmaske 24 erfolgt. Weitere Einzelheiten sind beispielsweise in der WO 2006/066744 A1 beschrieben, auf die in diesem Zusammenhang Bezug genommen wird.

Durch Hinterätzen können extrem scharfe Formteilkanten 28 bzw. Grate auftreten, die an der Spitze 14 erwünscht sind, an der Hinterkante 22 aber zum Hindernis bei der Ausführung der Stechbewegung werden können. Insbesondere kann während des Stechvorgangs, bei dem der Microsampler 10 innerhalb einer Führung beispielsweise eines aus vergleichsweise weichem Kunststoff bestehenden Magazins bewegt wird, ein unerwünschter Kanteneingriff und Materialabrieb auftreten.

Um derartige Probleme zu vermeiden, ist eine Lasernachbearbeitung zur Verrundung bzw. Entgratung der proximalen Formteilkanten 28 vorgesehen. Fig. 3 veranschaulicht eine Laserbearbeitungsvorrichtung 30 und den Verlauf der Laserstrahlführung auf dem Halteteil 18 des Microsamplers 10. Dabei wird mittels eines Lasers 32 und einer an sich bekannten Strahlführungseinrichtung 34 ein Laserstrahl 36 in einer mehrspurigen Bestrahlungsbahn 38 in mehreren aufeinanderfolgenden Bestrahlungsintervallen über das Halteteil 18 geführt, so dass die proximalen Formteilkanten durch den akkumulierten Laserenergieeintrag verrundet werden. Hierfür kann zweckmäßig ein Lasersystem mit einer geringen Strahlleistung (ca. 10W) eingesetzt werden, wie es beispielsweise als Beschriftungslaser unter der Bezeichnung TruMark von der Firma TRUMPF Lasertechnik erhältlich ist.

Ausgehend von einem von der Kante 22 beabstandeten Materialbereich des Halteteils 18 wird der Laserstrahl 36 in seitlich einander überlappenden Spuren 40 bis in einen kantennahen Bereich geführt. Das Halteteil 18 wird somit intervallweise mit abnehmendem seitlichem Versatz parallel zu den Formteilkanten 28 mit dem Laserstrahl 36 überstrichen, wobei die Bestrahlung beidseitig gleichzeitig oder nacheinander erfolgen kann. Dabei wird die Wärmeeinflusszone von der Materialstruktur her intervallweise bis in den Kantenbereich getrieben, wo durch den akkumulierten Energieeintrag eine Veränderung der Materialstruktur im Sinne der gewünschten Entgratung bzw. Kantenverrundung auftritt. Bei geringer Materialdicke etwa von weniger als 0,2 mm kann eine einseitige Bestrahlung genügen, um sowohl die dem Laserstrahl zugewandte Formteilkante als auch die abgewandte Kante in einem Arbeitsgang zu entschärfen.

Der Laserstrahl 36 wird mit einer Bahngeschwindigkeit von beispielsweise 20 mm/s über das beispielsweise nur 1 mm breite Halteteil 18 hinweg bewegt, so dass die einzelnen Bestrahlungsintervalle nur eine kurze Dauer von beispielsweise 50 ms haben. Zwischen den Bestrahlungsintervallen kann eine Wartezeit insbesondere aufgrund einer sukzessiven Strahlführung über eine Anordnung von mehreren Bauteilen liegen. Der Energieeintrag des Laserstrahls 36 in den einzelnen Bestrahlungsintervallen sollte unterhalb der Schwelle zu einem Materialabtrag eingestellt werden, so dass keine Schmelztropfen gebildet oder gar Strukturteile zerstört werden. Hierbei wird zweckmäßig die Anzahl der Bestrahlungsintervalle in Abhängigkeit von einem gewünschten Kantenmaß vorbestimmt. Beispielsweise können somit die Formteilkanten 28 mit einem Kantenradius von etwa 50 µm verrundet werden.

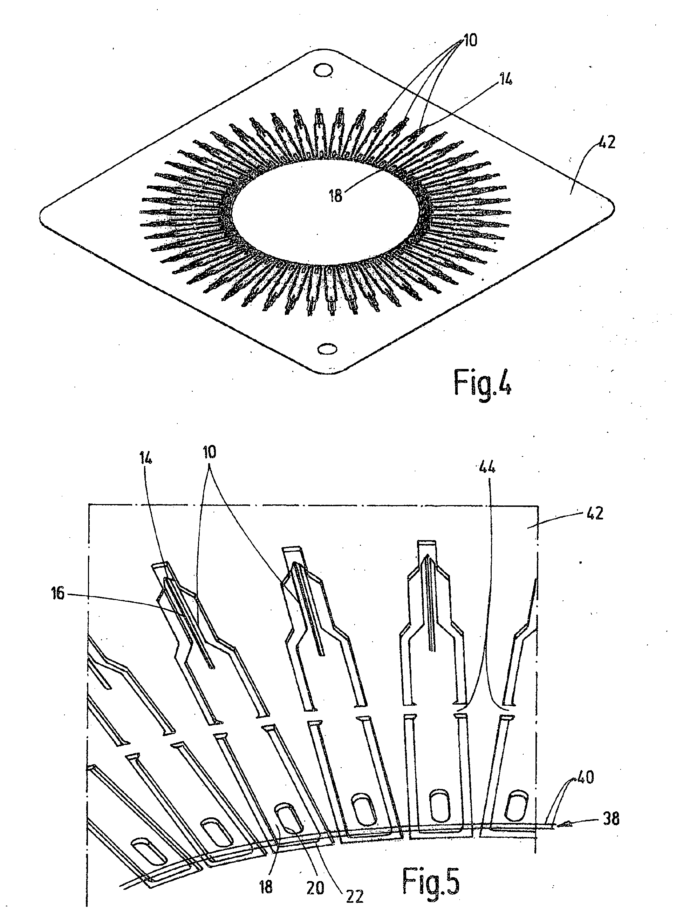

Wie in Fig. 4 gezeigt, kann bei dem Verfahrensschritt des Maskenätzens eine Vielzahl von Microsamplern 10 in einer gewünschten Anordnung erzeugt und für eine Magazinierung beispielsweise in einem kreisförmigen Scheibenmagazin bereitgestellt werden. Dabei werden die Microsampler 10 in sternförmiger Anordnung mit radial nach außen weisenden Spitzen 14 und auf einem Innenkreis liegenden Halteteilen 18 aus einer Blechplatte 42 gebildet.

Wie aus der Ausschnittvergrößerung nach Fig. 5 ersichtlich, kann durch entsprechendes Maskenlayout eine Materialtrennung entlang der Formteilkanten erfolgen, wobei die einzelnen Microsampler 10 in dem Ätzgitter über brechbare Materialbrücken 44 für die nachfolgende Laserbestrahlung noch in zusammenhängender Konfiguration gehalten werden. Anschließend wird der Laserstrahl 36 in konzentrischen Kreisen 40 mit abnehmendem Durchmesser über die kreisförmig koaxial angeordneten Halteteile 18 hinweg zu den Formteilkanten 22 hin geführt, um die gewünschte Kantenverrundung zu erreichen.

## Patentansprüche

1. Verfahren zum Herstellen von analytischen Hilfsmitteln zum Gewinnen von Körperflüssigkeit, insbesondere von Lanzetten oder Microsamplern, bei dem die Hilfsmittel als Formteil (10) aus einem metallischen Flachmaterial unter Materialtrennung an Formteilkanten (28) gebildet werden, **dadurch gekennzeichnet, dass** mindestens eine scharfkantige Formteilkante (28) durch Laserbestrahlung nachbearbeitet wird, wobei ein Laserstrahl (36) entlang einer Bestrahlungsbahn (38) in Bestrahlungsintervallen mehrfach über das Formteil (10) hinweg geführt wird und die Formteilkante (28) durch den akkumulierten Energieeintrag des Laserstrahls (36) verrundet wird, wobei der Laserstrahl (36) ausgehend von einem kantenfernen Materialbereich des Formteils (10) in einen kantennahen Bereich geführt wird.

2. Verfahren nach Anspruch 1, **dadurch gekennzeichnet, dass** der Laserstrahl (36) in seitlich einander überlappenden Spuren (40) geführt wird.

3. Verfahren nach Anspruch 1 oder 2, **dadurch gekennzeichnet, dass** der Laserstrahl (36) intervallweise mit abnehmendem seitlichen Versatz zur Formteilkante (28) geführt wird.

4. Verfahren nach einem der Ansprühe 1 bis 3, **dadurch gekennzeichnet, dass** die zu bearbeitenden Formteilkanten (28) einer Vielzahl von Formteilen (10) kreisförmig angeordnet werden, und dass der Laserstrahl (36) auf einer Spiralbahn oder in konzentrischen Kreisen über die Formteile hinweg zu den zu entgratenden Formteilkanten (28) hin geführt wird.

5. Verfahren nach einem der Ansprühe 1 bis 3, **dadurch gekennzeichnet, dass** die zu entgratenden Formteilkanten (28) einer Vielzahl von Formteilen (10) linear angeordnet werden, und dass die nebeneinander liegenden Formteile mit dem Laserstrahl (36) zeilenweise überstrichen werden.

6. Verfahren nach einem der Ansprühe 1 bis 5, **dadurch gekennzeichnet, dass** eine Vielzahl von Formteilen (10) in sternförmiger oder linearer Anordnung gegebenenfalls über Materialbrücken (44) zusammenhängend in einem einheitlichen Ätzvorgang aus einem Flachmaterialstück (42) gebildet werden.

7. Verfahren nach einem der Ansprühe 1 bis 6, **dadurch gekennzeichnet, dass** die Hilfsmittel durch Maskenätzen als Formteil (10) gebildet werden, und dass beim Maskenätzen der Formteile (10) unter der Einwirkung eines Ätzmittels durch Unterätzen einer Formteilmaske (24) eine gegebenenfalls gratbehaftete, scharfe Formteilkante (28) gebildet wird.

8. Verfahren nach einem der Ansprühe 1 bis 7, **dadurch gekennzeichnet, dass** die Formteile (10) mit einer scharfen Spitze (14) zum Einstechen in die Haut und gegebenenfalls einem Sammelkanal (16) für Körperflüssigkeit geformt werden.

9. Verfahren nach einem der Ansprühe 1 bis 8, **dadurch gekennzeichnet, dass** eine Formteilkante (28) in einer zur Antriebskopplung ausgebildeten Kopplungspartie der Formteile (10) verrundet wird.

10. Verfahren nach einem der Ansprühe 1 bis 9, **dadurch gekennzeichnet, dass** der Energieeintrag des Laserstrahls (36) in den einzelnen Bestrahlungsintervallen unterhalb der Schwelle zu einem Materialabtrag eingestellt wird.

11. Verfahren nach einem der Ansprühe 1 bis 10, **dadurch gekennzeichnet, dass** die Anzahl der Bestrahlungsintervalle nach Maßgabe eines gewünschten Kantenmaßes einer Formteilkante (28) vorbestimmt wird.

12. Verfahren nach einem der Ansprühe 1 bis 11, **dadurch gekennzeichnet, dass** die Formteilkante (28) mit einem Kantenradius von mehr als 30 µm, vorzugsweise etwa 50 µm verrundet wird.

13. Analytisches Hilfsmittel zum Gewinnen von Körperflüssigkeit, mit einem durch Maskenätzen aus einem metallischen Flachmaterial gebildeten Formteil (10), **dadurch gekennzeichnet, dass** mindestens eine Formteilkante (28) durch Laserbestrahlung nach einem Verfahren gemäß einem der vorhergehenden Ansprüche verrundet ist.

## Claims

1. A method for producing analytical aids for obtaining bodily fluid, in particular lancets or microsamplers, in which the aids are formed as a shaped part (10) from a flat metallic material by material separation on shaped part edges (28), **characterized in that** at least one sharp shaped part edge (28) is finished by laser irradiation, a laser beam (36) being guided repeatedly along over the shaped part (10) along an irradiation path (38) in irradiation intervals, and the shaped part edge (28) being rounded by the cumulative energy input of the laser beam (36), wherein the laser beam (36) is guided into a region near the edge starting from a material region of the shaped part (10) distant from the edge.

2. The method as claimed in claim 1, **characterized in that** the laser beam (36) is guided on tracks (40) laterally overlapping one another.

3. The method as claimed in claim 1 or 2, **characterized in that** the laser beam (36) is guided in intervals with decreasing lateral offset from the shaped part edge (28).

4. The method as claimed in one of claims 1 to 3, **characterized in that** the shaped part edges (28) to be processed, of a multiplicity of shaped parts (10), are arranged circularly, and **in that** the laser beam (36) is guided on a spiral path or in concentric circles over the shaped parts to the shaped part edges (28) to be deburred.

5. The method as claimed in one of claims 1 to 3, **characterized in that** the shaped part edges (28) to be deburred, of a multiplicity of shaped parts (10), are arranged linearly, and **in that** the shaped parts lying next to one another are scanned in lines with the laser beam (36).

6. The method as claimed in one of claims 1 to 5, **characterized in that** a multiplicity of shaped parts (10) are formed in an integrated etching process from a flat material piece (42) in a star-shaped or linear arrangement, optionally connected by material bridges (44).

7. The method as claimed in one of claims 1 to 6, **characterized in that** the aids are formed as a shaped part (10) by mask etching, and **in that** an optionally burred sharp shaped part edge (28) is formed during the mask etching of the shaped parts (10) by etching under a shaped part mask (24).

8. The method as claimed in one of claims 1 to 7, **characterized in that** the shaped parts (10) are formed with a sharp tip (14) for insertion into the skin and optionally a collecting channel (16) for bodily fluid.

9. The method as claimed in one of claims 1 to 8, **characterized in that** a shaped part edge (28) is rounded in a coupling portion of the shaped parts (10) formed for drive coupling.

10. The method as claimed in one of claims 1 to 9, **characterized in that** the energy input of the laser beam (36) in the individual irradiation intervals is set below the threshold for material ablation.

11. The method as claimed in one of claims 1 to 10, **characterized in that** the number of irradiation intervals is predetermined in accordance with a desired edge dimension of a shaped part edge (28).

12. The method as claimed in one of claims 1 to 11, **characterized in that** the shaped part edge (28) is rounded with an edge radius of more than 30 µm, preferably about 50 µm.

13. An analytical aid for obtaining bodily fluid, comprising a shaped part (10) formed from a flat metallic material by mask etching, **characterized in that** at least one shaped part edge (28) is rounded by laser irradiation according to a method as claimed in one of the preceding claims.

## Revendications

1. Procédé de fabrication d'accessoires d'analyse destinés au prélèvement d'un fluide corporel, notamment de lancettes ou de micro-échantillonneurs, dans lequel les accessoires sont formés sous la forme d'une pièce façonnée (10) à partir d'un matériau métallique plat par séparation de matière au niveau d'arêtes de pièce façonnée (28), **caractérisé en ce qu'**au moins une arête (28) de la pièce façonnée à arêtes vives est réusinée par irradiation au laser, un faisceau laser (36) passant à plusieurs reprises sur la pièce façonnée (10) le long d'un trajet d'irradiation (38) à des intervalles d'irradiation donnés et l'arête de pièce façonnée (28) étant arrondie par l'apport d'énergie accumulé du faisceau laser (36), le faisceau laser (36) étant amené d'une zone de matière de la pièce façonnée (10) éloignée de l'arête dans une zone proche de l'arête.

2. Procédé selon la revendication 1, **caractérisé en ce que** le faisceau laser (36) suit des tracés (40) se chevauchant latéralement.

3. Procédé selon la revendication 1 ou 2, **caractérisé en ce que** le faisceau laser (36) est déplacé, par intervalles, avec un décalage latéral décroissant par rapport à l'arête de pièce façonnée (28).

4. Procédé selon l'une des revendications 1 à 3, **caractérisé en ce que** les arêtes de pièce façonnée (28) à usiner d'une pluralité de pièces façonnées (10) sont disposées en cercle et **en ce que** le faisceau laser (36) est déplacé sur les pièces façonnées en suivant un trajet en spirale ou en cercles concentriques et en avançant vers les arêtes de pièce façonnée (28) à ébavurer.

5. Procédé selon l'une des revendications 1 à 3, **caractérisé en ce que** les arêtes de pièce façonnée (28) à ébavurer d'une pluralité de pièces façonnées (10) sont disposées de manière linéaire et **en ce que** les pièces façonnées placées les unes à côté des autres sont parcourues ligne par ligne par le faisceau laser (36).

6. Procédé selon l'une des revendications 1 à 5, **caractérisé en ce qu'**une pluralité de pièces façonnées (10), disposées en étoile ou de manière linéaire et tenues, le cas échéant, par des ponts de matière (44), est formée à partir d'une pièce de matériau plat (42) en un processus de gravure uniforme.

7. Procédé selon l'une des revendications 1 à 6, **caractérisé en ce que** les accessoires sont formés par gravure sous masque sous la forme d'une pièce façonnée (10) et **en ce qu'**une arête vive (28), le cas échéant présentant des bavures, de la pièce façonnée est formée lors de la gravure sous masque des pièces façonnées (10) sous l'action d'un agent de gravure par sous-gravure d'un masque (24) de pièce façonnée.

8. Procédé selon l'une des revendications 1 à 7, **caractérisé en ce que** les pièces façonnées (10) sont façonnées avec une pointe acérée (14) destinée à piquer la peau et, le cas échéant, un canal collecteur (16) pour un fluide corporel.

9. Procédé selon l'une des revendications 1 à 8, **caractérisé en ce qu'**une arête de pièce façonnée (28) est arrondie dans une partie d'accouplement des pièces façonnées (10) conçue pour l'accouplement à une commande.

10. Procédé selon l'une des revendications 1 à 9, **caractérisé en ce que** l'apport d'énergie du faisceau laser (36) dans les intervalles d'irradiation individuels est réglé sous le seuil permettant un enlèvement de matière.

11. Procédé selon l'une des revendications 1 à 10, **caractérisé en ce que** le nombre d'intervalles d'irradiation est prédéterminé en fonction d'une dimension d'arête souhaitée pour une arête de pièce façonnée (28).

12. Procédé selon l'une des revendications 1 à 11, **caractérisé en ce que** l'arête de pièce façonnée (28) est arrondie avec un rayon d'arête supérieur à 30 µm, de préférence d'environ 50 µm.

13. Accessoire d'analyse destiné au prélèvement d'un fluide corporel, comprenant une pièce façonnée (10) formée par gravure sous masque à partir d'un matériau métallique plat, **caractérisé en ce qu'**au moins une arête de pièce façonnée (28) est arrondie par irradiation au laser conformément à un procédé selon l'une des revendications précédentes.
